(19)

**Europäisches Patentamt**
**European Patent Office**
**Office européen des brevets**

(11) **EP 1 779 899 A1**

(12) **EUROPÄISCHE PATENTANMELDUNG**

(43) Veröffentlichungstag:
**02.05.2007 Patentblatt 2007/18**

(21) Anmeldenummer: **05026090.0**

(22) Anmeldetag: **30.11.2005**

(51) Int Cl.:
*A61Q 9/04* *(2006.01)*     *A61K 8/44* *(2006.01)*
*A61K 8/34* *(2006.01)*     *A61K 8/46* *(2006.01)*
*A61K 8/41* *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **23.06.2005 EP 05013593**

(71) Anmelder: **Tex-A-Tec AG**
**9630 Wattwil (CH)**

(72) Erfinder:
• **Marte, Walter**
**9631 Ulisbach/SG (CH)**

• **Meyer, Martin**
**8800 Thalwil (CH)**
• **Dutler, Hans**
**8053 Zürich (CH)**
• **Zimmermann, Michael**
**5033 Buchs (CH)**

(74) Vertreter: **Forstmeyer, Dietmar et al**
**BOETERS & LIECK**
**Oberanger 32**
**80331 München (DE)**

(54) **Zusammensetzung zur Depilierung**

(57)     Die vorliegende kosmetische Zusammensetzung zur Haarentfernung verwendet in Lösemitteln lösliche Aluminium- bzw. Zink-Komplexe der Thioglykolsäure zur Spaltung der Cystin-Schwefelbrücken, sowie tertiäre Amine als Solvolyse-Katalysatoren. Die Verwendung eines solchen Systems ermöglicht auch bei einem relativ niedrigen pH-Wert von 10,5 eine schnelle hautschonende Entfernung der Haare. Durch die Verwendung von Katalysatoren zur Mizellar- und Phasentransfer-Katalyse in Verbindung mit Mercaptoverbindungen kann eine weitere Verkürzung der Depilationszeit erzielt.

EP 1 779 899 A1

**Beschreibung**

*Hintergrund der Erfindung*

Die Erfindung betrifft ein chemisches Fiaarentfernungssystem aus einer Depilierungsfonnulierung kombiniert mit verschiedenen Applikationssystemen, die eine dem Haarwuchs angepasste minimierte Dosierung der Depilierungsmasse zulassen. Der Anwendungsbereich betrifft sämtliche mit Haar bewachsenen Körperteile inklusiv dem Intim- und Gesichtsbereich,

Die Zielsetzungen derartiger Depilierungssysteme liegen, dem kosmetischen Modetrend folgend, in der raschen, schonenden und schmerzfreien Haarentfernung, ohne Hautirritationen und Geruchsbelästigungen. Sehr wesentlich dabei ist, dass das Haarprotein möglichst rasch fragmentiert und dabei das Hautprotein, insbesondere das Stratum corneum, nicht angegriffen wird. Dies ist durch den unterschiedlichen Aufbau des Haar- und Hautproteins möglich. Das Haarprotein besteht aus $\alpha$-Keratin und Kollagen, wobei die $\alpha$-Helices über Disulfidbrücken miteinander vernetzt sind. Die Festigkeit des Haarproteins wird dadurch verstärkt, dass drei helicale Stränge einander umschlingen und eine Superhelix bilden, deren Zwischenräume Fette unterschiedlicher Zusammensetzung enthalten (z.B. freie Fettsäuren, Triglyceride, freies Cholesterol, Esterwachse) (A.L. Lehninger. D.L. Nelson, M.M. Cox, Prinzipien der Biochemie. Spektrum Akademischer Verlag Heidelberg Berlin Oxford, 1994). Der Anteil an Cystein zur Bildung des Cystins kann bis zu 18 % des Haarproteins betragen. Gemäss diesen Gegebenheiten besteht die Solvolyse des Haares in der reduktiven Spaltung des Cystins (Disulfidbrücke) mit der alkalisch katalysierten Peptid-Defibrillierung, die weitgehend diffusionskontrolliert abläuft. Da das Stratum Comeum als wesentliche Schutzschicht der Haut kein Cystin enthält, ist dessen Solvolysemechanismus deutlich unterschiedlich zum Haarprotein. Dies ermöglicht durch die geeignete Wahl der Depilierungsingredienzien eine selektive Fragmentierung des Haarproteins. Durch die Steuerung reaktionstechnischer Parameter des Haarsolvolyseprozesses in den verschiedenen Reaktionsschritten sowie den Einsatz neuer System gerechter Applikationssysteme kann den erwähnten Forderungen Folge geleistet werden.

*Stand der Technik*

Die heute im Handel erhältlichen Depilierungssysteme zeigen in ihrem Design eine grosse Vielfalt. Besonders häufig sind Cremen und Schäume anzutreffen, die sich in ihren chemischen Formulierungen und Applikationsformen nur unwesentlich unterscheiden. Typische Beispiele besagter Anwendungsformen sind: Creme, Spray, Gel, Gel-Creme, kaltes/warmes Wachs, Epilativnsgeräte, sowie Nass- und Trockenrasur für Beine, Bikinizonen, Achsel, Gesicht, Intimbereich, und Brusthaare.

Zur Verminderung der Geruchsbelästigung und zum Schutz gegen Bakterienbefall werden den Formulierungen Duftstoffe (z.B. Pfefferminze, Eucalyptus, Nerol, Farnesol, Rosenöl etc.) und Bakterizide (Eucalyptus, p-Hydroxybenzoesäure und deren Ester etc.) beigemischt. Weitere Ingredienzien der Depilierungscremen zur Quellungsverstärkung der Haare sind Hamstoff und dessen Derivate.

Andere Depilierungssysteme, deren Formulierung im Vergleich mit Cremen eine niedrigere Viskosität aufweisen, sind in Verpackungsformen wie Fläschchen, Tiegel, Roll-on, Tuben etc. abgepackt und werden in dünnen Schichten mittels eines Auftragsrollers, Schwammpolsters oder Tuches appliziert. Ein derartiges Beispiel ist Ex-Hair-Roll-on (Firma Medosan). Diese Systeme zeigen aus heutiger Sicht deutliche Vorteile im Vergleich mit Cremen und Schäumen, da die Kontaminationsgefahr durch die Depilierungsmasse mit Körperstellen wie Hände, Augen etc. und die Auftragsdosis der Depilierungsmasse weit geringer ist.

Allen diesen Systemen gemeinsam ist der sehr hohe pH-Wert, der mehrheitlich bei 13 und höher liegt, sowie die relativ hohe Konzentration der depilierend wirkenden Thioglykolsäure, die in den meisten Produkten verwendet wird und deren Konzentration bis zu 0.8 mol/l betragen kann. Die Thioglykolsäure wird in der Regel als Natrium-, Kalium- und/oder Calciumsalz, in Verbindung mit Natron- und Kalilauge sowie Calciumhydroxid eingesetzt. Infolge der nur begrenzt vorhandenen Biokompatibilität handelsüblicher Formulierungen treten Hautirritationen auf, die wiederum durch entsprechende Formulierungsingredienzien wie z.B. Kamillenextrakt (Azulen) und Amikaextrakt bekämpft werden.

Aufgrund des sehr hohen pH-Wertes der meisten Depilierungsformulierungen sind diese auch nicht im Intimbereich oder an anderen kritischen Körperstellen wie z.B. im Gesicht anwendbar. Weitere standardmässig eingesetzte Ingredienzien sind Verdickungskomponenten wie z.B. Fettalkohole, Fettsäuren, Sorbitol und Polysaccharide bzw, deren Derivate sowie Dispergatoren und Emulgatoren. Einige wichtige Hersteller solcher Depilierungssysteme sind: Gillette, Reckitt Benkiser, PILCA (GlaxoSmithKline), SNEA EPIL (Rufin), X EPIL (Stemmark), NAIR (Church & Dwight), und NADS.

*Aufgabe der Erfindung*

Eine Aufgabe der Erfindung besteht darin, eine schnell wirkende, schonende und geruchsarme Zusammensetzung zur schmerzfreien Entfernung von Haaren bereitzustellen, ohne Hautreizungen zu verursachen. Eine weitere Aufgabe besteht darin, ein Applikations-System für die vorstehende Depilierungsformulierung bereitzustellen, das gleichzeitig eine das Haar entfernende Peelingfunktion übernimmt. Bei der Konstruktion des Applikationssystems soll die Gefahr einer unerwünschten Kontamination, z.B. der Hände, durch die Depilierungsmasse minimiert werden und eine umweltgerechte Entsorgung des benutzten Depilierungssystemes möglich sein. Eine weitere Aufgabe der Erfindung besteht darin, eine Zusammensetzung bereitzustellen, mit der die Depilierungszeit reduziert werden kann.

### *Lösung der Aufgabe*

Die Aufgabe wird durch eine kosmetische Zusammensetzung zur Entfernung von Haaren gelöst, umfassend ein Entfettungssystem mit mindestens einer fettlösenden Verbindung, die eine M-Zahl nach Godfrey von 1 bis 18 besitzt, und ein Solvolyse-System mit mindestens einer Mercapto-Verbindung.

Die Entfernung der Haare verläuft vorzugsweise in drei Schritten:

Der erste umfasst ein Solvatisieren bzw. Lösen und gegebenenfalls Emulgieren des das Haar schützenden Fettes (= Hydrolipidfilm), mit Hilfe des in der Depilierungsmasse enthaltenen **"Entfettungssystems".** Dadurch wird der Angriff durch die die Solvolyse des Haares auslösenden Wirkstoffe begünstigt.

Der zweite Schritt umfasst den vorzugsweise katalytisch geführten Solvolyseprozess des Haares, der in Wasserstoffbrücken und Disulfidbrücken (Cystin) spaltende sowie untergeordnet in Peptid spaltende Reaktionen unterteilt werden kann. Die entsprechenden Ingredienzien sind im **"Solvolysesystem"** der Depilierungsformulierung zusammengefasst.

Der dritte, optionale Schritt beinhaltet die Entfernung der Depilierungsmasse mit den Haarfragmenten von der Haut durch einen **Peelingprozess** als optionaler Bestandteil des "Depilierungssystems".

### *Beschreibung der Erfindung im Detail*

### 1. **Entfettungs-System: Entfernung und Mobilisierung der Fettschicht**

Die Haarentfernung erfolgt insbesondere durch Spaltung der Cystin-Schwefelbrücken. Damit die für die Spaltung der Schwefelbrücken erforderlichen Ingredienzien an ihren Wirkungsort gelangen können, ist die Entfernung der das Haar umgebenden Hydrolipidschicht (= Schutzschicht aus Fett, die das Haar umgibt) vorteilhaft. Die Entfettung der Haare ist also der erste Schritt und wird in vorteilhafter Weise durch den Einsatz weitgehend in Wasser unlöslicher, Fettsorbierender Verbindungen in Kombination mit wasserlöslichen Dispergatoren bzw. Emulgatoren und niedermolekularen Fettlösemitteln, vorzugsweise mit Ether-Alkoholen durchgeführt.

Das Entfettungssystem kann zum Beispiel bis zu drei Bestandteile umfassen, nämlich ein Fettlösemittel, ein Fettsorptionsmittel und einen Emulgator. Die einzelnen Bestandteile haben folgende Funktionen: Das Fettlösemittel (Punkt 1.1.) löst das Fett aus dem Haar und mobilisiert, d.h. transportiert es in die Öl-Phase der "Öl in Wasser" - Emulsion. Die Öl-Phase dieser Emulsion umfasst mindestens ein Fett-Sorptionsmittel (Punkt 1.2.). Aufgrund des Umstandes, daß das Haarfett durch das Fettlösemittel solubilisiert bzw. gelöst und somit beweglich ist, kommt es zu einer Sorption, d.h. teilweisen Immobilisierung, des Haarfettes in der Öl-Phase bzw. den Tensidmizellen der Emulsion, welche die Fett-Sorptionsmittel umfaßt.

Die Pettlösemittel ermöglichen folglich den Transport des Haarfettes vom Haar in die Öl-Phase der Emulsion, in welcher sie teilweise immobilisiert werden. Durch die Immobilisierung der Haarfette in der Öl-Phase bzw. den Tensidmizellen der Emulsion wird das Verteilungsgleichgewicht zwischen an Haar sorbierten Fetten und in der wässrigen Lösemittel-Phase gelösten Fetten zugunsten des in der Öl-Phase sorbierten Anteiles verschoben.

Die eingesetzten Dispergatoren bzw. Emulgatoren sind grenzflächenaktive Mittel, welche unter anderem auch die Stabilisierung der Öl-Phase in der wässrigen Phase und somit die Bildung der Emulsion bewirken.

### 1.1. **Fettlösemittel**

Als **Fettlösemittel,** deren Aufgabe es ist, Haar schützende Fette vom Haar zu solubilxsieren bzw. zu lösen und zu mobilisieren, d.h. den Transport in die Öl-Phase der Emulsion bzw. in die Tensidmizellen zu ermöglichen, werden vorzugsweise polare, protische Lösemittel eingesetzt. Bevorzugte Beispiele hierfür sind Alkohole mit 1, 2, 3, 4 oder 5 Kohlenstoffatomen, und deren Ether.

Die eingesetzten Fettlöser, deren M-Zahlen im Bereich von 1 - 18 liegen, zeichnen sich sowohl durch ihre gute Wasser- als auch Fettlöslichkeit aus, Die M-Zahlencharakterisierung von Lösemitteln basiert gemäss den Arbeiten von Godfrey auf deren Affinität für "Öl-ähnliche Substanzen" (N.B. Godfrey, ChemTech, June 1972, S. 359).

Allgemein sind Fettlösemittel im Sinne dieser Erfindung polare, protische, wasser-lösliche, flüssige Verbindungen.

Typische Vertreter sind Alkohole mit 1, 2, 3,4. oder 5 Kohlenstoffatomen, und Ether-Alkohole, niedermolekulare, wasserlösliche Ethylenoxid-Homopolymere (zum Beispiel Pluriol E mit einem Molekulargewicht von 102 bis 2.000 der Fa. BASF) und Propylenoxid-Homo-polymere (zum Beispiel Pluriol P mit einem Molekulargewicht von 134 bis 600 der Fa. BASF) und deren Derivate, niedermolekulare, statistische und wasserlösliche Ethylenoxid-und Propylenoxid-Copolymere und deren Derivate, niedermolekulare, wasserlösliche Ethylenoxid- und Propylenoxid-Blockcopolymerisate (zum Beispiel Pluronic PE mit einem Molekulargewicht von 1.000 bis 4.000 und Pluronic RPE mit einem Molekulargewicht von 2.000 bis 4,000) und deren Derivate, sowie Ether-Alkohole wie zum Beispiel Ethoxyethanol (Ethoxyglykol), Propoxyethanol (Propoxyglykol), Butoxyethanol (Butoxyglykol). Butylacetat und Glycerintriacetat. Als sehr geeignet haben sich Pluronic, insbesondere Pluronic PE 4300 (Firma BASF), und Butoxyglykol erwiesen.

Die Molekulargewichte der Fettlösemittel liegen vorzugsweise im Bereich von 100 bis 4.000, bevorzugt von 2.000 bis 4.000, stärker bevorzugt von 1.000 bis 4.000.

Es kann sowohl ein Fettlösemittel allein als auch eine Kombination von zwei, drei oder mehreren derselben eingesetzt werden.

Die Einsatzmenge dieser polaren, protischen Lösemittel liegen bevorzugt im Bereich von 1 - 15 Gew.-%, vorzugsweise 3 - 6 Gew.-%, bezogen auf gesamte kosmetische Zusammensetzung.

### 1.2. Fett-sorbierende Verbindungen

Die durch die Lösemittel mobilisierten Fette werden vorzugsweise entsprechend ihrer Löslichkeit in der wässrigen Lösemittel-Phase von den optional einsetzbaren, Fett sorbierenden **Verbindungen** des "Entfettungssystems" immobilisiert, wodurch weiteres Fett in der wässrigen Phase gelöst werden kann. Allgemein sind Fett-sorbierende Verbindungen im Sinne dieser Erfindung vorzugsweise Tensidmizellen, die optional auch weitgehend wasser-unlösliche, fettartige Verbindungen enthalten, d.h. Verbindungen, deren Löslichkeit weniger als 1 % beträgt; daher werden sie als Öl-Phase in Wasser emulgiert. Die Fett-sorbienden Verbindungen stellen im Wesentlichen die Öl-Phase der "Öl in Wasser" - Emulsion dar.

Als **Fett-sorbierende** und - vorzugsweise gleichzeitig die Depilierungsmasse verdickende - Komponenten können - neben Mineralölen - Fettalkohole, Fettsäuren, Alkyl- und Arylethoxilaten sowie Fettamide mit jeweils 12, 13, 14, 15, 16, 17, 18, 19, 20, 21, 22, 23, oder 24 Kohlenstoffatomen eingesetzt werden. Diese Verbindungen haben die Aufgabe. das bereits durch die Pettlösemittel gelöste Fett in der wässrigen Phase der "Öl in Wasser"-Emulsion zu immobilisieren, damit durch die Fettlöser neues Fett gelöst werden kann.

Typische Vertreter dieser Substanzklassen sind Cetylalkohol, Stearinsäure und deren Veresterungsderivate wie z.B. Octyl- und Cetylpalmitate und Triglyceride, sowie ethoxylierte und propoxylierte Fettalkohole mit jeweils 12, 13, 14, 15, 16, 17, 18, 19, 20, 21, 22, 23, oder 24 Kohlenstoffatomen im Fettalkohol; die Zahl der Ethylenoxid-bzw. Propylenoxid-Einheiten in den ethoxylierten bzw. propoxylierten Fettalkoholen beträgt 1 bis 10, vorzugsweise 1-5.

Neben den bereits erwähnten Substanzen sind Silizium organische Verbindungen wie z.B. Phenyldimethicone, Cetyldimethicone oder Cyclomethicone ebenfalls sehr gut geeignet. Vorzugsweise eingesetzte Produkte sind Cetyl- und Cetearylalkohol.

Es kann sowohl ein Fettsorptionsmittel allein als auch eine Kombination von zwei, drei oder mehreren derselben eingesetzt werden.

Die Fett-sorbierenden Substanzen werden vorzugsweise im Konzentrationsbereich von 2-15 Gew.-% eingesetzt, vorzugsweise von 5 - 10 Gew.-%, bezogen auf die gesamte kosmetische Zusammensetzung.

**1.3.** Als **Dispergatoren bzw. Emulgatoren,** die Bestandteile des Entfettungssystems sein können, können grenzflächenaktive Substanzen mit HLB-Werten zwischen 7 und 18 eingesetzt werden (Hydrophilic-Lipophilic-Balance, siehe H. Sonntag, Lehrbuch der Kolloidwissenschaft, VEB Deutscher Verlag der Wissenschaften Berlin 1977).

Besonders geeignete Vertreter dieser Substanzklasse sind Laurylsulfat, Laurethsulfat, Span 20 (Sorbitanlaurat), Tween 20 (ethoxyliertes Sorbitanlaurat), Brij 58 (Polyoxyethylen-(20)-cetylether), Renex 720 (ethoxylierter $C_{13}$ - $C_{15}$ - Alkohol) und Laureth-23. Speziell geeignet ist eine Kombination bestehend aus $^2/_3$ Laurethsulfat und $^1/_3$ Laurylethoxylat, jeweils bezogen auf das Gewicht.

Es kann sowohl eine grenzflächenaktive Substanz allein als auch eine Kombination von zwei, drei oder mehreren derselben eingesetzt werden.

Die Gesamteinsatzmenge der grenzflächenaktiven Substanzen liegt bevorzugt im Bereich von 2-10 Gew.-%, vorzugsweise 4 - 6 % bezogen auf die gesamte kosmetische Zusammensetzung.

### 1.4. Verdickungsmittel für die wässrige Phase

Darüber hinaus können Verdickungsmittel für die wässrige Phase der "Öl in Wasser"-Emulsion eingesetzt werden. Damit kann die Viskosität der kosmetischen Zusammensetzung gesteigert und ihre Handhabung erleichtert werden.

Als typische Vertreter für Verdickungsmittel der wässrigen Phase kommen in Betracht: Natrium-Alginate, insbesondere Alginate EHV (CHT-Tübingen), Polyacrylate, zum Beispiel Dynasorb der Fa. Stockhausen, sowie Cellulose-Derivate, zum Beispiel Methocel 311 der Fa. Dow Chemical Company oder Bermocell E 230-FQ der Fa. Akzo Nobel und assoziativ wirkende Verdickungsmittel wie z.B, Polyurethane, die mit Alkyl-Aryl-Polyglykolethern kombiniert sind, insbesondere Borchi Gel L 75 N der Firma Borchers GmbH.

Es kann sowohl ein Verdickungsmittel allein als auch eine Kombination von zwei, drei oder mehreren derselben eingesetzt werden.

## 2. Solvolysesystem

### 2.1. Lösemittel-lösliche Metallkomplexe von Mercapto-Verbindungen

Die Solvolyse des Haares, als zweiter Schritt, wird vornehmlich durch die Tertiär- und Sekundärstruktur spaltenden Depilierungsingredienzien bewerkstelligt ("Solvolysesystem"). Ein Gesichtspunkt dieser Erfindung betrifft die Verwendung von ein- oder zweibasigen Mercapto-Verbindungen.

Als zweibasige Mercapto-Verbindungen kommen insbesondere Metallkomplexe der Thioglykolsäure, d.h. das Dianion der Thioglykolsäure, in Betracht, die teilweise in polaren, protischen Lösemitteln, zum Beispiel Ethanol, löslich sind. Insbesondere sind diese Metallkomplexe in den unter Punkt 1.1. aufgeführten Fettlösemitteln teilweise löslich,

Als gleichwertige Alternative zur Thioglykolsäure und deren Salzen können auch einbasige Mercapto-Verbindungen, wie z.B. Mercaptoethanol oder Mercaptoglycerin bzw. deren Salze, eingesetzt werden. Bevorzugte Salze des Mercaptoethanols bzw. des Mercaptoglycerins sind das Lithium-, Natrium-, Kalium-, Magnesium-, Calcium-, Aluminium-, Titan-, Zinn-, und Zinksalz. Diese weisen ebenfalls eine vergleichbare oder bessere Löslichkeit in polaren, protischen Lösemitteln auf wie die Salze der Thioglykolsäure.

Es kann sowohl eine Mercapto-Verbindung allein als auch eine Kombination von zwei, drei oder mehreren derselben eingesetzt werden; insbesondere können alle Salze der Thioglykolsäure mit Mercaptoethanol und/oder Mercaptoglycerin in Kombination verwendet werden.

Als Cystin spaltende Ingredienzien können neben den vorstehend genannten Natrium-, Kalium- oder Calcium-Thioglykolaten erfindungsgemäss zumindest teilweise in Alkohol lösliche Metall-Thioglykolsäure-Komplexe der A- und B-Kationen, wie Aluminium- oder Zink-Thioglykolsäure-Salze (siehe hierzu: A. Ackermann, W. Jugelt, H.H. Möbius, H.D. Suschke, G. Werner, Elektrolytgleichgewichte und Elektrochemie, Verlag Chemie Weitlheim, 1974, Seiten 221 - 223) verwendet werden.

Damit die Spaltungsreagenzien eine Solvolyse des Haares durchführen können ist, wie vorstehend angegeben, zunächst eine teilweise Entfernung der das Haar umgebenden Schutzschicht aus Fett erforderlich. Diese Entfernung erfolgt üblicherweise durch das vorstehend erwähnte Entfettungssystem. Die teilweise Entfernung der das Haar umgebenden Schutzschicht aus Fett ist dann ausreichend, wenn eine Mizellar- und/oder Phasentransferkatalyse verwendet wird, da diese durch die Fettschicht des Haares hindurch wirken kann.

Die vorliegenden Erfinder haben in unerwarteter Weise gefunden, daß Aluminium- bzw. Zink-Komplexe der Thioglykolsäure die Spaltung der Cystin-Schwefelbrücken beschleunigen. Während die Natrium- und Kaliumsalze der Thioglykolsäure in erster Linie wasserlöslich, nicht jedoch in den Lösemitteln löslich sind, zeichnen sich die in dieser Erfindung bevorzugt eingesetzten Aluminium- und Zink-Komplexe der Thioglykolsäure durch eine deutliche Löslichkeit in den Lösemitteln, z.B. in den Alkoholen aus, die auch als Fettlösemittel (siehe Punkt 1.1.) eingesetzt werden können.

Die Thioglykolsäure-Salze des Aluminiums und des Zinks grenzen sich von den Natrium-, Kalium- und Calciumsalzen dadurch ab, dass letztere praktisch vollständig dissoziiert sind, d.h. als Salze vorliegen und daher in den unter Punkt 1.1. aufgeführten Fettlösemitteln nicht löslich sind. Demgegenüber liegen die Aluminium- bzw. Zink-Verbindungen der Thioglykolsäure nicht als Salze vor, sondern sind undissoziierte Komplexe, die eine deutliche Löslichkeit in den Fattlösemitteln im Sinne dieser Erfindung (Punkt 1.1.), beispielsweise Ethanol, aufweisen.

Dadurch wird bereits eine Spaltung der Cystin-Schwefelbrücken des Haares eingeleitet, während der Entfettungsprozess noch andauert. Auf diese Weise wird eine besonders schnelle Haarentfernung ermöglicht, indem der Entfettungsprozess und der Solvolyseprozess zumindest teilweise gleichzeitig ablaufen. Diese Beobachtung trifft auch auf die einbasigen Mercapto-Verbindungen Mercaptoethanol und Mercaptoglycerin bzw. deren Salze zu. Somit werden Depilierungszeiten von 3 Minuten möglich.

Vorzugsweise eingesetzte Thioglykolsäurekomplexe beinhalten Aluminium und Titan als Beispiele für das A-Kation bzw. Zink und Zinn für das B-Kation. Die Komplexe der Thioglykolsäure werden in einer Konzentration von 0,2 M bis 0,8 M, bevorzugt von 0,2 M bis 0,4 M, insbesondere bevorzugt etwa 0,3 M eingesetzt.

Gegebenenfalls können zusätzlich Alkali-Salze der Thioglykolsäure eingesetzt werden.

Als weitere Substanzen zur Spaltung von Cystin-Disulfidbrücken kommen Mercaptoethanol und Mercaptoglycerin in Betracht, wobei die verwendeten Konzentrationen ebenfalls im Bereich von 0,2 M bis 0,8 M, bevorzugt von 0,2 M bis 0,4 M, insbesondere bevorzugt etwa 0,3 M liegen. Das zur Salz- bzw. Ionenpaarbildung befähigte Mercaptoethanol besitzt einen pKs-Wert von 9,37, womit bei einem pH-Wert von 11 circa 98% des eingesetzten Mercaptoethanols in seiner deprotonierten Form vorliegen. Damit reduziert sich dessen dermale Toxizität wesentlich gegenüber der protonierten Form.

## 2.2. Mizellar- bzw. Phasentransferkatalyse

Ein wesentlicher Gesichtspunkt dieser Erfindung betrifft die optionale Verwendung von Mizellar- bzw. Phasentransfer-Katalysatoren, wobei vorzugsweise quaternäre Ammoniumverbindungen eingesetzt werden. Grundsätzlich ist bei den quaternären Ammoniumverbindungen zwischen niedermolekularen Verbindungen, wie zum Beispiel Tetrabutylammoniumchlorid, und hochmolekularen, d.h. polymeren Verbindungen, wie zum Beispiel Polyquaternium 28, einem Polymerisat aus Vinylpyrrolidon und Methacrylamidopropyl-trimethylammoniumchlorid, zu unterscheiden. Während die niedermolekularen quaternären Ammoniumverbindungen die klassischen Phasentransfer-Katalysatoren darstellen und aufgrund ihrer geringen Neigung zur gegenseitigen Assoziation weniger zur Mizell-Bildung neigen, besitzen die tensidartigen und polymeren quaternären Ammoniumverbindungen eine ausgeprägte Neigung, Mizellen in der Wasserphase zu bilden. Andererseits ist die Neigung der hochmolekularen quaternären Ammoniumverbindungen, als klassische Phasentransfer-Katalysoren die Phasengrenze zwischen der wässrigen und der organischen Phase zu überschreiten, tendentiell geringer ausgeprägt als bei den entsprechenden niedermolekularen Verbindungen. Darüber hinaus hängt die Bildung der Mizellen von der Konzentration der eingesetzten quaternären Ammoniumverbindung ab. Bevorzugte quaternäre Ammoniumverbindungen sind solche mit tensidartigem Charakter, Bei der Mizellar-Katalyse muß die sog. "kritische Mizellen-Konzentration (CMC)" des quaternären Ammoniumsalzes in jedem Fall überschritten werden. Daher hängt es von der Konstitution und der Konzentration der eingesetzten quaternären Ammoniumverbindung(en) ab, in welchem Ausmaß die Mizellbildung in der Wasserphase auftritt, und folglich auch, in welchen Ausmaß die Phasentransfer-Katalyse und/oder die Mizellar-Katalyse auftreten. Je nach verwendeter Verbindung kann daher die Phasentransfer-Katalyse oder die Mizellar-Katalyse dominieren. Die quaternären Ammoniumverbindungen zeigen eine gute Wasserlöslichkeit, sind aber auch in polaren protischen und aprotischen Lösungsmitteln, wie z,B. Ethanol oder Dioxan, teilweise löslich. Beide Arten der Katalyse werden nachstehend näher erläutert.

Eine logische Trennung zwischen Mizellar- und Phasentransfer-Katalyse ist zwar aufgrund kinetischer Messungen möglich, für die Ausführbarkeit der vorliegenden Erfindung jedoch nicht nötig. Vielmehr können beide Arten der Katalyse im vorliegenden System nebeneinander ablaufen.

### 2.2.1. Mizellarkatalyse

Die **Mizellarkatalyse** folgt in vielen Fällen dem Prinzip einfacher Enzymmodellc. Dies lässt sich durch einige Ähnlichkeiten belegen:

- Die Grösse von Mizellen und globulären Proteinen ist ähnlich.
- Sowohl Mizellen als auch Proteine können geeignete Moleküle solubilisieren.
- Es gibt viele chemische Reaktionen in mizellaren Lösungen, deren Kinetik einen ähnlichen Verlauf haben wie enzymkatalysierte Reaktionen.

Das hierfür gültige allgemeine Reaktionsmodell ist in Abbildung 1 dargestellt:

$$\text{Mizf} + \text{S} \underset{}{\overset{K}{\rightleftharpoons}} \text{MizS}$$

$$\downarrow k_w \qquad\qquad\qquad\qquad \downarrow k_{miz}$$

$$P \qquad\qquad\qquad\qquad\qquad P$$

$$Mizf^{\oplus} \; + \; \begin{matrix} ^{\ominus}S\text{-}R \\ ^{\ominus}OH \end{matrix} \quad \underset{\longleftarrow}{\overset{K}{\longrightarrow}} \quad Miz\Big]^{(x+y)\,\oplus} (^{\ominus}S-R)_x \, (^{\ominus}OH)_y$$

$$\Big\downarrow K_w = \text{„5 min"} \qquad\qquad \Big\downarrow K_{miz} = \text{„3 min"}$$

$$P \qquad\qquad\qquad P$$

Abb. 1 Reaktionsschema einer einfachen Mizellar-Katalyse

Unter dem Begriff Substrat versteht man im Fall der Mizellar-Katalyse die reaktiven Zentren des Haares, d.h. die SS-Cystinbrücken, die Wasserstoffbrücken-Bindungen, und die Peptid-bindungen; die Reaktanden umfassen Mercapto-Verbindungen und das Alkali.

Die Reaktanden, welche die Solvolyse des Haares bewirken, (abgekürzt S), zum Beispiel das negative geladene Hydroxid-Ion, Thioglykolat-Anion, Mercaptoglycerin oder Mercaptoethanol assoziieren in einem schnellen Gleichgewichtsschritt mit einer Mizelle (Mizf), die aus dem vorzugsweise rensidartigen und/oder hochmolekularen positiv geladenen quaternären Ammoniumsalz besteht, zum Mizellkomplex (MizS). Vorzugsweise weist mindestens ein Rest des quaternären Ammoniumsalzes 12 bis **24** Kohlenstoffatome auf, vorzugsweise weist genau ein Rest des quaternären Ammoniumsalzes 12, 14, 16, 18, 20, 22 oder 24 Kohlenstoffatome auf; die anderen drei Reste bestehen beispielsweise aus Methylgruppen. Die Reaktivitäten von S in der freien und der assoziierten Form sind durch die Geschwindigkeitskonstanten $k_w$ bzw. $k_{miz}$ definiert. Aufgrund der Mizellbildung kann die Konzentration der Mercapto-Verbindungen an der Oberfläche der Mizelle weitaus höher sein, als sie im Normalfall wäre, d.h, falls die Mercapto-Verbindungen in wässriger ösung vorläge. Durch die hohe Konzentration der Mercapto-Verbindungen S (OH⁻, R-S⁻) an der Mizelloberfläche ergeben sich durch die Sorption der Mizellen auf dem Haar höhere Reaktionsgeschwindigkeiten bezüglich der Haarsolvolyse im Vergleich zu einem Solvolyse-System ohne Mizellen, Damit kann wiederum die Depilationszeit verkürzt und die Hautreizung vermieden werden; ebenso kann die Konzentration der Mercapto-Verbindung gesenkt und somit die Geruchsbelästigung reduziert werden.

Die Beladung der Mizelloberfläche mit den Reaktanden für die Solvolyse (z.B. mit Mercapto-Verbindungen) erfolgt in einem schnellen, der Reaktion vorgelagerten Gleichgewichtsschritt, wobei die Reaktion, d,h. die Spaltung der Wasserstoffbrücken-Bindungen und der SS-CystinBrücken mehrheitlich nach nullter Ordnung abläuft.

### 2.2.2. Phasentransfer-Katalyse

Das Prinzip der **Phasentransfer·Katalyse** besteht darin, ionische Substanzen aus der Wasserphase in das organische Medium (in diesem Fall in die Fettphase) zu überführen, Hierzu sehr geeignet sind quaternäre Verbindungen [Q⊕X⁻], deren Salze bzw. Ionenpaare sowohl eine gute Fettlöslichkeit als auch eine gute Wasserlöslichkeit besitzen. In der Fettphase bedarf es keiner Solvatation des Salzes bzw. Ionenpaars.

Das allgemeine Reaktionsprinzip ist in Abbildung 2 dargestellt; nicht berücksichtigt sind jedoch weitere, der schematischen Reaktion vor- und nachgelagerte proteolytische Gleichgewichtsreaktionen.

wässrige Phase     $Q^+X^- \; + \; S^- \;\rightleftharpoons\; [Q^+S^-] \; + \; X^-$

Phasengrenze

organische Phase     $[Q^+X^-] \; + \; BS \;\rightleftharpoons\; [Q^+S^-] \; + \; BX$

Abb. 2 Allgemeines Reaktionsschema der Phasentransferkatalyse

[Q⊕X⁻] entspricht der vorzugsweise eingesetzten quaternären, positiv geladenen Verbindung, vorzugsweise einem quaternären Ammoniumsalz, zum Beispiel Tetrabutylammoniumchlorid, wobei Q⁺ der quaternären Verbindung, in diesem Beispiel dem Tetrabutylammonium, und X⁻ dem Gegenion, in diesem Beispiel dem Chlorid, entspricht. [S] entspricht einer depilierend wirkenden Komponente bzw. dem konjugierten Anion (z.B. OH⁻, R-S⁻, deprotoniertes Mercaptoethanol); und [Q⊕S⁻] entspricht dem sich bildenden von der Fettphase des Haares extrahierten Reaktionsprodukt aus dem quaternären Ammoniumsalz und dem anionischen Reaktanden für die Solvolyse. Das extrahierte Reaktionsprodukt zeigt die depilierende Wirkung (Reaktionsprodukt BS) unter Rückbildung des eingesetzten quaternären Salzes [Q⊕X⁻] (Ionenpaar) gemäss Abbildung 2. BX steht für das Substrat, d.h. im allgemeinen Wasserstoffbrücken-Bindungen und SS-Cystin-Bindungen; dieses reagiert mit dem QS zum BS, das für gespaltene Wasserstoffbrücken-Bindungen, Peptid-Bindungen und SS-Cysteinbrücken steht, unter Rückbildung des urspünglichen quaternären Ammoniumsalzes [Q⊕X⁻].

Die im Rahmen der Mizellar- bzw. Phasentransfer-Katalyse einzusetzenden reduktiv wirkenden Mercapto-Verbindungen, zeichnen sich dadurch aus, dass sie in der Lage sind, Salze bzw. Ionenpaare mit den eingesetzten Mizell- oder Phasentransferkatalysatoren, zu bilden. Als hierfür geeignete Substanzen sind neben der Thioglykolsäure und deren Salze, Mercaptoethanol und Mercaptogycerin zu erwähnen.

### 2.2.3. Quaternäre Ammoniumverbindungen für die Mizellar- bzw. Phasentransfer-Katalyse

Vorzugsweise eingesetzte quaternäre Verbindungen für die Mizellar- und Phasentransfer-Katalyse zeichnen sich durch vierfach substituierte Ammoniumverbindungen aus, wobei die Substituenten sowohl aliphatische als auch aromatische Strukturen aufweisen können. Dar-über hinaus kann der quaternäre Stickstoff selbst Bestandteil eines aromatischen Ringsystems sein, z.B. eines Pyridin- bzw. Pyridinium-Rings.

Bei den aliphatischen Substituenten handelt es sich zum Beispiel um Kohlenwasserstoffketten von $C_1$ - $C_{24}$, bevorzugt von $C_1$ - $C_{14}$ und OH-terminierte Ethylenoxid- und Propylenoxidreste. Der Ethoxylierungs- bzw. Propoxylierungsgrad bewegt sich zwischen 1 und 30, bezogen auf die Ethylenoxid- und Propylenoxid-Einheiten im Rest. Typische Vertreter sind Tetrabutylammoniumchlorid, Methyltrioctylammoniumchlorid, Fettamine ($C_4$ - $C_{24}$), die mit Dimethylsulfat quaternisiert werden, quaternisierte Fettamine der allgemeinen Struktur $(C_4\text{-}C_{24})NMe_3^+$, wie zum Beispiel N,N,N-Trimethyl-N-dodecyl-ammoniumchlorid, N,N,N-Trimethyl-N-tetradecyl-ammoniumchlorid (N-Cetyl-N,N,N-trimethylammoniumchlorid), N,N,N-Trimethyl-N-hexadecyl-ammoniumchlorid, N,N,N-Trimethyl-N-octadecyl-ammoniumchlorid, N,N,N-Trimethyl-N-eicosanyl-ammvniumchlorid, N,N,N-Trimethyl-N-docosanyl-ammoniumchlorid, N,N,N-Trimethyl-N-tetracosanyl-ammoniumchlorid; DERIPHAT® 160 C (Natrium-N-lauryl-beta-iminodipropionat), Deriphat 151 C (N-Cocobeta-aminopropionsäure der Firma carechemicals), DERIPHAT® 154L (Dinatrium Tallow-Iminodipropionat der Firma carechemicals), sowie Cocobis(2-hydroxyethyl)methylammoniumchlorid.

Während die bisher genannten Substituenten homologe Fettkohlenwasserstoffe, Ethylenoxid-oder Propylenoxidreste darstellen, sind auch Aromaten enthaltende Substituenten einsetzbar wie z.B. N-[3-(paranonylphenoxy)-2-hydroxypropyl] N,N,N-trimethylammoniumchlorid.

Eine weitere Möglichkeit ist der Einsatz von polymeren quaternären Ammoniumverbindungen, wobei die quaternären Gruppen auf einem Rückgrat des Polymers kovalent gebunden sind. Beispiele hierfür sind Copolymere aus Vinylpyrrolidon und Methacrylamidopropyl-trimethylammoniumchlorid (Polyquaternium-28 der Fa. GAF) oder quaternisierte Copolymere aus Vinylpyrrolidon und Dimethylaminoethylmethacrylat (Polyquaternium-11).

Quaternäre Ammoniumsalze mit aromatischen bzw. heterocyclischen Strukturen weisen bevorzugt Phenyl-, oder Pyridiniumstrukturen auf. Der aliphatische Substituent ist in der Regel eine Kohlenwasserstoffkette von $C_1$ - $C_{24}$, bevorzugt $C_1$ - $C_{14}$, insbesondere ein Substituent mit 1, 2, 3, 4, 6, 8, 10, 1,2 14, 16, 18, 20, 22 oder 24 Kohlenstoffatomen.

Beispiele hierfür sind: Trimethylphenylammoniumchlorid bzw. -bromid, Methylpyridiniumchlorid, Cetylpyridiniumchlorid und 1-Dodecylpyridiniumbromid.

Als negative geladene Gegenionen der quaternären Ammoniumverbindungen kommen vorzugsweise Fluorid, Chlorid. Bromid, Iodid, Sulfat, Hydrogensulfat oder Hydroxid in Betracht, bevorzugt Chlorid und Sulfat.

Es kann sowohl eine quaternäre Ammoniumverbindung allein als auch eine Kombination von zwei, drei oder mehreren derselben eingesetzt werden; insbesondere können tensidartige und polymere quaternäre Ammoniumverbindungen in Kombination eingesetzt werden.

Die Einsatzmengen der Mizellar- bzw. Phasentransfer-Katalysatoren sind abhängig von deren Konstitution und Mizellbildungskonzentration (CMC) und liegen vorzugsweise im Bereich von 0.001 - 0.2 mol/l, bevorzugt im Bereich von 0.01 - 0.1 mol/l, jeweils bezogen auf die gesamte kosmetische Zusammensetzung.

Bevorzugt eingesetzte reduktive Mercapto-Verbindungen sind Thioglykolsäure, bzw. deren Salze, Mercaptoethanol und Mercaptoglycerin. Diese werden vorzugsweise in Konzentrationen von 0.2 - 0.8 mol/l, bevorzugt 0.2 bis 0.4 mol/l, jeweils bezogen auf die gesamte kosmetische Zusammensetzung, eingesetzt. Falls Mizellar- und/oder Phasentransfer-Katalysatoren eingesetzt werden, kommen als Reaktanden zur Solvolyse des Haares bevorzugt Mercaptoethanol und Mercaptoglycerin in Betracht.

### 2.3. Tertiäre Amine als Katalysatoren: DABCO und DBU

Zusätzlich kann das Solvolyse-System mindestens ein tertiäres Amin umfassen; durch derartige Amine wird die Solvolyse von Haar und insbesondere die Spaltung von Wasserstoffbrücken und untergeordnet auch von Peptiden katalysiert.

Allgemein sind tertiäre Amine im Sinne dieser Erfindung vorzugsweise cyclische Amine mit mindestens einer, vorzugsweise zwei tertiären Amin-Funktionen und mindestens einem Ring, die nicht unter Bedingungen der Depilierung hydrolysieren, d.h. bei einem pH von kleiner oder gleich 11,5 und einer Temperatur von kleiner oder gleich 37°C. Insbesondere sind solche Amine bevorzugt, die der Struktur $NR^1R^2R^3N$ entsprechen, wobei $R^1$, $R^2$, und $R^3$ jeweils unabhängig geradkettige oder verzweigte, gesättigte oder ungesättigte, substituierte oder unsubstituierte, zweiwertige Kohlenwasserstoff-Gruppen mit 2, 3, 4, 5, 6 oder 7 Kohlenstoffatomen darstellen, wobei jeweils zwei dieser Gruppen zusammen einen Ring bilden können.

Als sehr effizient haben sich hierbei die Substanzen 1,4-Diazabicyclo[2,2,2]octan (DABCO), 1,8-Diazabicyclo[5.4.0]undec-7-en (DBU), 1,4-Diezabicyclo[4.3.0]non-5-en und Triethanolamin $N(CH_2CH_2OH)_3$ erwiesen.

Es kann sowohl ein tertiäres Amin allein als auch eine Kombination von zwei, drei oder mehreren derselben eingesetzt werden.

### 2.4. Schonung der Haut durch niedrigen pH-Wert: Puffersystem

Durch die Verwendung der tertiären und quaternären Amine als Solvolyse-Katalysatvren ist es möglich, die Depilierung bei pH-Werten von 9 - 11.5 vorzugsweise bei pH 10 - 11, anstelle des sonst üblichen pH-Wertes von 13 und höher, durchzuführen. Dadurch kann die Entfernung der Haare in sehr kurzer Zeit wesentlich hautschonender durchgeführt werden; trotz des erniedrigten pH-Wertes im Vergleich zu herkömmlichen Depilierungsmassen wird durch die Katalysatoren eine ausreichende Geschwindigkeit für die Solvolyse des Haares bzw. der Haardefibrillierung erzielt.

Die Einstellung des pH-Wertes der kosmetischen Zusammensetzung erfolgt zum Beispiel durch den Einsatz von puffersystemen wie z.B. Kaliumhydrogencarbonat / Kaliumcarbonat für einen pH-Bereich von 9,5 bis 11,0, dessen Puffer-Maximum bei etwa 10.5 liegt, sowie Siliziumdioxid / Natronlauge (Wasserglas) für einen pH-Bereich von 11 - 12, dessen Puffer-Maximum bei etwa 11 liegt. Die Einsatzmenge der korrespondierenden Säure-Basenpaare ist vorzugsweise so zu wählen, dass in der kosmetischen Zusammensetzung dieser Erfindung eine Pufferkapazität von 0.02 - 0.06 mol/l, vorzugsweise 0.04 - 0.05 mol/l vorherrscht.

Durch die Verwendung tertiärer und quaternärer Amine wie DABCO, DBU, Tetrabutylammoniumhydroxid und Polyquatemium-28 sowie durch den Einsatz bei pH 10 bis 11,5, bevorzugt bei etwa 10,5, bewegen sich die Depilierungszeiten je nach Haarstärke und Fettungsgrad der Haare zwischen 3 und 5 Minuten, üblicherweise zwischen 3 und 4 Minuten.

Darüber hinaus kann durch die Verwendung des erfindungsgemäßen Katalysator- und Solvolysesysems die Menge der einzusetzenden Mercapto-Verbindung, zum Beispiel Thioglykolsäure und Mercaptoethanol und Mercaptoglycerin, bzw. von deren Salzen bzw. Derivaten reduziert werden, so dass durch die im Vergleich zu herkömmlichen Depilierungsmitteln geringe Konzentration an Mercapto-Verbindung eine merkliche Verminderung der Geruchsbelästigung erreicht werden kann.

### 2.5. Weitere Bestandteile des Solvolyse-Systems

Ferner kann die kosmetische Zusammensetzung mindestens ein reduzierendes Endiol oder Endiolat oder deren Derivate umfassen. Beispiele derartiger Endiole oder Endiolate sind Ascorbinsäure, Ketozucker wie Fruchtzucker, sowie Acetoin und Adepoin. Als stark reduktiv wirkende Endiolate, auch Reduktone genannt, sind Ketozucker (z.B. Fructose), Acetoin und Adepoin hervorzuheben. Die Endiol-Verbindungen können in einer Konzentration von 0,2 M bis 0,5 M, bevorzugt 0,2 M bis 0,3 M eingesetzt werden.

Eine weitere und sehr effiziente Möglichkeit, die Cystinspaltungsgeschwindigkeit zu erhöhen, ist durch den Einsatz von Dithiothreitol gegeben, da das Reaktionsgleichgewicht der Disufidspaltung zu Gunsten des Dithiothreitol-Produktes verschoben wird. Dithiothreitol kann in einer Konzentration von 0,2 M bis 0,8 M, bevorzugt von 0,2 M bis 0,4 M eingesetzt werden.

### 3. Beispiele und experimenteller Teil

Die Basisinformationen zur Entwicklung der erfindungsgemässen Depilierungsformulierung wurden sowohl aus kinetischen Daten der Haarsolvolyse als auch aus den Haarbruchdaten gewonnen. Als Prüflinge dienten Methanol gereinigte, ca. 300 mm lange Frauenhaare.

Zur Messung der Solvolysekinetik wurde das Haar in die entsprechende Depilierungsflotte getaucht, mit einem Vorspanngewicht von 300 - 350 mN belastet und die in Folge der Haarfragmentierung abnehmende Vorspannkraft in der zeitlichen Abfolge gemessen. Zur Erfassung der Bruchzeit wurde das Haar mit einer konstanten Vorspannkraft von 295 mN belastet und bis zum Bruch in die entsprechende Depilierungsflotte getaucht. Das der Arbeitshypothese zu Grunde gelegte Reaktionsschema (Abb, 1) beschreibt eine Folgereaktion pseudo erster Ordnung, mit einem in der ersten Reaktionsstufe, d,h. zwischen A und B mit den Geschwindigkeitskonstanten $k_1$ und $k_1$, auftretenden Gleichgewichtsschritt, der hauptsächlich der Disulfidspaltung zu zuordnen ist. Der zweite, irreversible Reaktionsschritt von B nach C mit der Geschwindigkeitskonstante $k_2$ entspricht der diffusionskontrollierten Wasserstoffbrücken- und der ebenfalls möglichen Peptidspaltung.

$$A \underset{k_{-1}}{\overset{k_1}{\rightleftharpoons}} B \xrightarrow{k_2} C$$

Abb.1 Reaktionschema der Haarsolvolyse (Cystin- und Wasserstoffbrücken spaltende, die Sekundär- und Tertiärstrukturen des Haares auflösenden Reaktionsstufen)

Als Zielgrössen zur Beschreibung der Wirkungsweisen der Depilierungsingredienzien werden die Reaktionskonstanten des ersten und zweiten Reaktionsschrittes (Abb. 1) sowie die Haarbruchzeit ermittelt und normiert. Die Bestwerte (grösste Reaktionskonstante und geringste Bruchzeit) entsprechen in der normierten Skala der Abbildung 2 Eins und die schlechtesten Werte Null. Durch die Zusammenführung der normierten Zielfunktionswerte der drei Zielgrössen zu unimodalen Funktionswerten $Z_i$ und die anschliessende semiempirische Modellierung wird eine die Wirkungsweisen der Depilierungsingredienzien beschreibende Funktion erhalten.

Eine weitere wesentliche Information ist aus der Abbildung 2 zu ersehen. Sie zeigt die Isolinien der normierten Zielgrösse (Depilierungsgeschwindigkeit) in Abhängigkeit des pH-Wertes und der Methanolkonzentration bei konstanter Thioglykolsäure- und DABCO-Konzentration (Abb. 2).

Abb. 2. Isolinien der normierten Depilierungsgeschwindigkeit in Abhängigkeit des pH-

Wertes und der Methanolkonzentration:

Abszisse: pH-Wert;
Ordinate: Methanolkonzentration in mol/l;
Konstante Werte: 0,3 M Kalium-Thioglykolat und Konzentration des tertiären
Amins 0.025 M DABCO.

Die zur Depilierung günstigsten Reaktionsbedingungen liegen gemäss Abbildung 2 bei ca. pH 10 und 3 mol/l Methanol.

Eine weitere wichtige Information ist der Abbildung 3 zu entnehmen. Sie zeigt die Isolinien der normierten Zielgrösse in Abhängigkeit des pH-Wertes und der DABCO-Konzentration mit den bestmöglichen Reaktionsbedingungen bei pH 10 - 10.5 und 0.02 - 0.03 mol/l DABCO. Die Konzentration des Kalium-Thioglykolats beträgt konstant 0.3 mol/l und die Methanol-Konzentration konstant 2 mol/l. Man erkennt, daß eine Konzentration von DABCO von 0,025 mol/l und ein pH-Wert von ca. 10,2 zu optimalen Depilierungs-Geschwindigkeit führt. Dennoch wird die Depilierung bei einem pH-Wert von etwa 10.5 durchgeführt, der auch der maximalen Pufferkapazität des Systems Kaliumhydrogencarbonat / Kaliumcarbonat entspricht, um stets die Funktionstüchtigkeit zu gewährleisten.

Abb. 3 Isolinien der normierten Depilierungs-Geschwindigkeit in Abhängigkeit des

pH-Wertes und der DABCO-Konzentration
Abszisse: pH-Wert
Ordinate: Konzentration von DABCO in mol/l
Konstante Werte: 0,3 M Kalium-Thioglykolat
und 2 M Methanol
Zur Messung der Solvolysekinetilt wurde das Haar in die entsprechende Depilierungsflotte getaucht, mit einem Vorspanngewicht von 300 - 350 mN belastet und die in Folge der Haarfragmentierung abnehmende Vorspannkraft in der zeitlichen Abfolge gemessen. Die Erfassung der Haarbruchzeit erfolgte bei konstanter Vorspannkraft von 295 mN und variablen Konzentrationen der Depilierungsingredienzien.

## 4. Handschuh als Peelingsystem

Zur Applikation der Depilierungsmasse dient erfindungsgemäss vorzugsweise ein "Handschuh", dessen eine Seite mit der Depilierungsmasse beschichtet ist und die Rückseite aufgrund der rauen Oberfläche als Peelinghandschuh zur Entfernung der Depilierungsmasse und der Haare verwendet wird. Die Fertigung eines derartigen Handschuhs ist äusserst kostengünstig, da die Handschuhe aus einem mehrlagigen Paket konfektioniert werden können. Die oberste Schicht des dreilagigen Paketes besteht zum Beispiel aus einem mit der Depilierungsmasse beschichteten Faservlies, die Mittelschicht ist beispielsweise eine Polyethylenfolie als Kontaminierungsbamere und die letzte entspricht zum Beispiel der Peelingschicht die aus einem rauhen Gewebe, Gewirk oder Vlies gefertigt ist. Zur Handschuhkonfektionierung wird die beschichtete, dreilagige, bis zu 2500 mm breite Warenbahn in ca. 150 mm breite Einzelbahnen geschnitten und an den Rändern vernäht bzw. verschweisst. Anschliessend werden die Einzelbahnen in 150 mm lange Einzelstücke geschnitten und nurmehr einseitig vernäht oder verschweisst, so dass ein Handschuh entsteht, dessen eine Seite die Depilierungsmasse trägt und die andere zur Reinigung (peeling) verwendet werden kann. In anderen Fällen ist es sinnvoll, die Vernähung bzw. Verschweissung des dreilagigen Applikationssysems bereits vor der Beschichtung durchzuführen.
Eine zweite Möglichkeit besteht in der Verwendung einer mit Klebstoff beschichteten Kunststofffolie in deren Beschichtungsmasse die zuvor erwähnten Depilierschemikalien eingearbeitet sind. Die beschichtete Seite der Folie wird mit einer zweiten Folie unmittelbar nach dem Beschichtungsprozess abgedeckt und gelangt als Warenbahn in die Konfektionierung. Die Haatentfernung mit der beschichteten Klebfolie erfolgt durch die Entfernung der Schutzfolie und Anpressung der beschichteten Folienseite an die entsprechende, zu enthaarende Körperstelle. Nach einer Einwirkzeit von 3 - 8 Minuten wird die Folie abgezogen und mit ihr die in Auflösung befindlichen Haare. Diese Art der Haarentfernung stellt eine Kombination aus Depilierung und Epilierung dar.

Beide erwähnten Applikationsformen zeichnen sich durch ihre einfache, und kostengünstige Herstellung aus und sind ausserdem umweltfreundlich zu entsorgen.

**Beispiel 1 Depilierungsformulierung und Applikationssystem**

Die in der nachfolgend aufgeführten Tabelle aufgelisteten **Entfettungs**chemikalien werden bis zur Schmelze erhitzt und vermischt und anschliessend in der Hälfte des zur Verfügung stehenden Wassers emulgiert. Die im **Solvolyse**system aufgeführten Chemikalien werden in einer wasservorlage gelöst, um anschliessend die zwischenzeitlich abgekühlte "Entfettungsemulsion" in der "Solvolyselösung" zu emulgieren. Nach erfolgter Emulgierung wird das restliche Wasser bis zu einem Volumen von einem Liter zugegeben.

Nun wird die Depilierungsformulierung mittels eines Beschichtungsprozesses auf die Vliesseite (Vliesstärke 1.5 mm) einer dreischichtigen Warenbahn (Vlies/Polyethylenfolie/Peelinggewirk) appliziert, die dann mit einer Schutzfolie überzogen wird. Die Beschichtungsauflage beträgt 500 g/m$^2$. Mit dieser Beschichtungsauflage kann pro "Handschuh" die Fläche eines Beines depiliert werden.

**Beispiel 1: Rezeptur der Depilierungsmasse**

| Funktionssystem | Ingredienzien | Funktion | Konzentration |
|---|---|---|---|
| **Entfettungs-System** | Cetylalkohol | Verdickung und Fettsorbierung | 30.0 g/l |
| | Laurylsulfat | Emulgator anionisch | 5.0 g/l |
| | Inbetin 180 (Kolb AG) | Emulgator nichtionogen | 5.0 g/l |
| | Pluronic PE 4300 (BASF) | Fettsolubilisierung | 5.0 g/l |
| | 2-Butoxyglykol | Fettlöser | 5.0 g/l |
| | Ethanol | Fottlöser | 30.0 g/l |
| **Solvolyse-System** | Kalium-Thioglykolat | Cyscinspaltung | 0.4 mol/l |
| | DABCO | Katalysator der Haarsolvolyse | 0.05 mol/l |
| | SiO$_2$/NaOH (Wasserglas) | pH-Wert Einstellung auf 11 | 0.5 mol/l |
| **Verdickungsmittel** | Alginat EHV (CHT-Tübingen) | Verdicker der Wasserphase | 14.0 g/l |

Das Peelinggewirk, als Reinigungsschicht des Handschuhs, ist aus groben Polyekhylenfasern gefertigt, um so die stark fragmentierten Haare, inklusiv der Depilierungsmasse zu entfemen.

Die auf diese Weise hergestellte Depilierungsformulierung zeichnet sich durch ihre geringe Geruchsbelästigung, den niedrigen pH-Wert von 11 sowie durch die kurze Depilierungszeit von 4 Minuten aus. Aufgrund des niedrigen pH-Wertes wurden auch bei Unterlassung einer Depilierungsnachbehandlung keine Hautinitationen festgestellt.

**Beispiel 2 Depilierungsformulierung und Applikation**

Die nachfolgend aufgeführte Formulierung entspricht einer Depilierungscreme, die im Schaumverfahren auf die Haut übertragen wird. Nach drei Minuten Einwirkzeit kann die Creme mittels eines nassen Peelinggewebes abgewaschen werden.

Die Herstellung der Creme erfolgt indem die Komponenten des Etfettungssystems (Imbentin, Glycerin, Pluriol und Pluronic) vermischt und auf ca. 50 °C aufgeheizt werden. Das Entfettungskomposit wird anschliessend im bereits hergestellten wässrigen Solvolysesystem emulgiert. Nach Fertigstellung der Emulsion wird das vorgemischte Verdickungssystem während ca. 15 Minuten eingerührt. Zum Schluss erfolgt die pH-Einstellung auf Ph 10.5.

**Beispiel 2: Rezeptur der Depilierungsmasse**

| Funktionssystem | Ingredienzien | Funktion | Konzentration |
|---|---|---|---|
| **Entfettungs-System** | Inbetin AG/124/15 (Kolb AG) | Emulgator nichtionogen | 5.0 g/l |
| | Glycerin | Lösungsmittel | 10.0 g/l |
| | Pluriol E 200 (BASF) | Fettsolubilisierungs- und Lösungsmittel | 5 g/l |
| | Pluronic PE 4300 (BASF) | Fettsolubilisierung und Lösungsmittel | 7 g/l |
| **Solvolyse-System** | Mercaptoethanol | Cystinspaltung | 0.4 mol/l |
| | Gafquat HS 100 (GAF) Polyquaternium 28 | Mizellkatalyse | 50 g/l |
| | DABCO | Katalysator der Haarsolvolyse | 0.1 mol/l |
| | Harnstoff | Haarquellungsmittel | 30 g/l |
| | Kaliumkarbonat | pH-Wert Einstellung auf 10.5 | 7 g/l |
| **Verdickungsmittel** | Prisulon CR-P450 (CHT) | Acrylatverdicker | 70.0 g/l |
| | Magnesiumhydroxid | basisches Pigment | 22 g/l |

Die Depilierungsveisuche zeigten, dass mit der angegebenen Formulierung, deren pH-Wert 10,5 beträgt und eine Mercaptoethanol-Konzentration von 0,4 mol/l aufweist, Depilierungszeiten von drei Minuten zu realisieren sind, ohne das üblicherweise vorhandene Gefahrenpotential durch eine sehr hohe Alkalikonzentration, die üblicherweise zu pH-Werten von 13 führt.

**Patentansprüche**

1. Kosmetische Zusammensetzung zur Entfernung von Haaren umfassend

    - ein Entfettungssystem mit mindestens einer zumindest teilweise in Wasser löslichen, fettlösenden Verbindung, die eine M-Zahl nach Godfrey von 1 bis 18 besitzt, und
    - ein Solvolyse-System mit mindestens einer Mercapto-Verbindung.

2. Kosmetische Zusammensetzung nach Anspruch 1, wobei die fettlösende Verbindung ausgewählt ist aus:

    - Alkoholen mit 1, 2, 3, 4, oder 5 Kohlenstoffatomen,
    - wasserlöslichen Ethylenoxid-Homopolymeren, Propylenoxid-Homopolymeren und deren Derivaten,
    - wasserlöslichen, statistischen Ethylenoxid- und Propylenoxid-Copolymeren und deren Derivaten,
    - wasserlöslichen Ethylenoxid- und Propylenoxid-Blockcopolymerisaten und deren Derivaten,
    - Ether-Alkoholen wie Ethoxyethanol (Ethoxyglykol), Propoxyethanol (Propoxyglykol), Butoxyethanol (Butoxyglykol), oder
    - Butylacetat und Glycerintriacetat.

3. Kosmetische Zusammensetzung nach Anspruch 1 oder 2, wobei die fettlösende Verbindung aus wasserlöslichen Ethylenoxid- und/oder Propylenoxid-Blockcopolymerisaten, bevorzugt Pluriol E, Pluriol P, Pluronic PE, Pluronic RPE, (alle von der Fa. BASF), insbesondere bevorzugt Pluronic PE 4300 (Fa. BASF) ausgewählt wird.

4. Kosmetische Zusammensetzung nach einem der vorstehenden Ansprüche, wobei die Zusammensetzung zusätzlich mindestens eine das Fett sorbierende Verbindung umfaßt.

5. Kosmetische Zusammensetzung nach Anspruch 4, wobei die das Fett sorbierende Verbindung wasserunlöslich ist und vorzugsweise ausgewählt wird aus:

    - Mineralölen,

- Fettalkoholen, Fettsäuren und Fettamiden jeweils unabhängig voneinander mit 12, 13, 14, 15, 16, 17, 18, 19, 20, 21, 22, 23, oder 24 Kohlenstoffatomen, wie Cetylalkohol, Stearinsäure, und deren Veresterungsprodukten,
- Triglyceriden,
- ethoxylierten und propoxylierten Fettalkoholen jeweils unabhängig voneinander mit 12, 13, 14, 15, 16, 17, 18, 19, 20, 21, 22, 23, oder 24 Kohlenstoffatomen im Fettalkohol sowie unabhängig voneinander mit 1 bis 10, vorzugsweise 1 bis 5 Ethylenoxid- bzw. Propylenoxid-Einheiten, oder
- siliciumorganischen Verbindungen.

6. Kosmetische Zusammensetzung nach Anspruch 4 oder 5, wobei die das Fett sorbierende Verbindung aus Octylpalmitat, Cetylpalmitat, Phenyldimethicone, Cetyldimethicone und Cyclomethicone ausgewählt ist.

7. Kosmetische Zusammensetzung nach einem der vorstehenden Ansprüche, wobei die Mercapto-Verbindung ausgewählt wird aus Thioglykolsäure und deren Salzen, insbesondere Natrium-, Kalium-, Calcium-, Aluminium-, Titan-, Zinn- oder ZinkSalzen der Thioglykolsäure, aus Dithiothreitol, aus Mercaptoethanol und/oder aus Mercaptoglycerin.

8. Kosmetische Zusammensetzung nach einem der vorstehenden Ansprüche, wobei die Zusammensetzung mindestens ein reduzierendes Endiol oder dessen Derivat, einen Ketozucker, Acetoin oder Adepoin umfaßt.

9. Kosmetische Zusammensetzung nach einem der vorstehenden Ansprüche, wobei das Solvolyse-System mindestens ein tertiäres Amin, insbesondere 1,4-Diaza-bicyclo[2.2.2]octan (DABCO), 1,8-Diazabicyclo[5.4,0]undec-7-en (DBU), 1,4-Diazabicyclo[4.3.0]non-5-en und/oder Triethanolamin umfaßt.

10. Kosmetische Zusammensetzung nach einem der vorstehenden Ansprüche, wobei das Solvolyse-System mindestens ein quaternäres Ammoniumsalz, insbesondere Tetrabutylammoniumchlorid oder ein Copolymer aus Vinylpyrrolidon und Methacrylamidopropyl-trimethylammoniumchlorid umfaßt.

11. Kosmetische Zusammensetzung nach einem der vorstehenden Ansprüche, wobei die Zusammensetzung ein Puffer-System umfaßt, und einen pH-Wert von 9 bis 12, vorzugsweise von 10 bis 11, besonders bevorzugt von etwa 10,5 aufweist.

12. Kosmetische Zusammensetzung nach Anspruch 11, wobei das Puffer-System

- Kaliumhydrogencarbonat / Kaliumcarbonat für einen pH-Wert von etwa 10,5, und/oder
- Siliciumdioxid / Natronlauge für einen pH-Wert von etwa 11 umfaßt.

13. Kosmetische Zusammensetzung nach einem der vorstehenden Ansprüche, wobei die Zusammensetzung mindestens ein grenzflächenaktives Mittel mit einem HLB-Wert von 7 bis 18 umfaßt.

14. Kosmetische Zusammensetzung nach Anspruch 13, wobei das grenzflächenaktive Mittel Laurylsulfat, Laurethsulfat, Span 20, Tween 20, Brij 58, Renex 720, Laureth-23, Inbetin 180, insbesondere bevorzugt eine Mischung aus 2/3 Laurethsulfat und 1/3 Laurylethoxylat, jeweils bezogen auf das Gewicht, umfaßt.

15. Kosmetische Zusammensetzung nach einem der vorstehenden Ansprüche, wobei die Zusammensetzung ein Verdickungsmittel umfaßt.

16. Kosmetische Zusammensetzung nach Anspruch 15, wobei das Verdickungsmittel aus Cetylalkohol, Natrium-Alginaten, Polyacrylaten, Cellulose-Derivaten und/oder Polyurethanen, optional in Kombination mit einem oder mehreren Alkyl-Aryl-Polyglykolethem, ausgewählt wird.

17. Verwendung einer kosmetischen Zusammensetzung nach einem vorgehenden Ansprüche zur Entfernung von Haaren.

18. Folienanordnung zur Entfernung von Haaren, mit einer Trägerfolie, wobei die Trägefolie

a) auf einer Seite mit einer kosmetischen Zusammensetzung nach einem der Ansprüche 1 bis 16 beschichtet ist.

19. Folienanordnung nach Anspruch 18, wobei die Schicht a) zusätzlich einen Klebstoff aufweist.

**20.** Folienanordnung nach Anspruch 18 oder Anspruch 19, wobei auf der Schicht a) eine abziehbare Schutzfolie b) angeordnet ist.

**21.** Folienanordnung in Form eines Handschuhs, dessen eine Oberfläche eine Trägerfolie mit einer kosmetischen Zusammensetzung nach einem der Ansprüche 1 bis 16 aufweist und dessen andere Oberfläche mit einer rauhen Oberfläche versehen ist.

**Europäisches Patentamt**

## EUROPÄISCHER RECHERCHENBERICHT

Nummer der Anmeldung

EP 05 02 6090

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | GB 2 367 749 A (* RECKITT BENCKISER FRANCE) 17. April 2002 (2002-04-17) * Seite 1, Zeilen 5-12; Beispiele 1-4 * * Seite 1, Zeilen 20,21 * ----- | 1-21 | INV. A61Q9/04 A61K8/44 A61K8/34 A61K8/46 A61K8/41 |
| X | US 2004/219118 A1 (SLAVTCHEFF CRAIG STEPHEN ET AL) 4. November 2004 (2004-11-04) * Absatz [0023]; Tabelle III * ----- | 1-21 | |
| X | EP 0 928 608 A (AJINOMOTO CO., INC) 14. Juli 1999 (1999-07-14) * Beispiel 44 * ----- | 1-21 | |
| X | WO 01/00144 A (LION CORPORATION; MITAMURA, JOJI; ONUKI, TAKESHI; NOGUCHI, MUTSUMI) 4. Januar 2001 (2001-01-04) * Beispiele 19,20 * ----- | 1-21 | |
| X | US 2003/175333 A1 (SHEFER ADI ET AL) 18. September 2003 (2003-09-18) * Beispiel 4; Tabelle 11 * ----- | 1-21 | RECHERCHIERTE SACHGEBIETE (IPC) A61K |
| X | US 2002/146380 A1 (NAMBU HIROMI ET AL) 10. Oktober 2002 (2002-10-10) * Beispiele 1-7 * ----- | 1-21 | |
| X | WO 2004/014179 A (RECKITT BENCKISER LIMITED) 19. Februar 2004 (2004-02-19) * Ansprüche; Abbildungen 3-5; Beispiele * ----- | 1-21 | |

Der vorliegende Recherchenbericht wurde für alle Patentansprüche erstellt

| Recherchenort | Abschlußdatum der Recherche | Prüfer |
|---|---|---|
| München | 13. März 2007 | Miller, Bernhard |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **A.L. LEHNINGER ; D.L. NELSON ; M.M. COX.** Prinzipien der Biochemie. Spektrum Akademischer Verlag, 1994
- **N.B. GODFREY.** Öl-ähnliche Substanzen. *ChemTech,* Juni 1972, 359
- **H. SONNTAG.** Lehrbuch der Kolloidwissenschaft. VEB Deutscher Verlag, 1977
- **A. ACKERMANN ; W. JUGELT ; H.H. MÖBIUS ; H.D. SUSCHKE ; G. WERNER.** Elektrolytgleichgewichte und Elektrochemie. Verlag Chemie, 1974, 221-223